Europäisches Patentamt

European Patent Office

Office européen des brevets

(11) Publication number: **0 369 664 B1**

# EUROPEAN PATENT SPECIFICATION

(45) Date of publication of patent specification: **23.02.94**  (51) Int. Cl.⁵: **C07K 7/10**, A61K 37/43, C12N 15/16

(21) Application number: **89311481.9**

(22) Date of filing: **06.11.89**

(54) **Fish CRF.**

(30) Priority: **14.11.88 US 270149**

(43) Date of publication of application:
**23.05.90 Bulletin 90/21**

(45) Publication of the grant of the patent:
**23.02.94 Bulletin 94/08**

(84) Designated Contracting States:
**AT BE CH DE ES FR GB GR IT LI LU NL SE**

(56) References cited:
**EP-A- 0 122 798**

(73) Proprietor: **THE SALK INSTITUTE FOR BIO-LOGICAL STUDIES**
**10010 North Torrey Pines Road**
**La Jolla California 92037(US)**

(72) Inventor: **Lederis, Karl P.**
**1515 Cavanaugh Palce NW**
**Calgary Alberta T2L 0M8(CA)**
Inventor: **Okawara, Yuji**
**17-24 Hongo-Cho**
**Numazu Shizuoka 410(JP)**
Inventor: **Richter, Dietmar**
**Suederfeldstrasse 44**
**D-2000 Hamburg 54(JP)**

(74) Representative: **Lawrence, Malcolm Graham et al**
**Hepworth, Lawrence & Bryer**
**2nd Floor, Gate House South**
**Westgate Road**
**Harlow Essex CM20 1JN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention is directed to a peptide hormone and to methods for treatment of animals using such a peptide. More specifically, the invention relates to the hentetracontapeptide CRF of the fish species Catostomus commersoni, to compositions containing such CRF and to methods of treatment using such CRF.

Experimental and clinical observations have supported the concept that the hypothalamus plays a key role in the regulation of adenohypophysial corticotropic cells secretory functions. Over 25 years ago, Guillemin, Rosenberg and Saffran and Schally independently demonstrated the presence of factors in hypothalamus which would increase the rate of ACTH secretion by the pituitary gland incubated in vitro or maintained in an organ culture. None of the secretagogs characterized met the criteria expected of a physiologic corticotropin releasing factor (CRF) until ovine CRF (oCRF) was characterized in 1981 and, as disclosed in U.S. Patent No. 4,415,558, was found to have the formula:

$$\text{H-Ser-Gln-Glu-Pro-Pro-Ile-Ser-Leu-Asp-Leu-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-Ala-NH}_2.$$

Sauvagine is a 40-residue, amidated generally similar peptide which was isolated from the skin of the South American frog Phyllomedusa sauvagei. It was characterized by Erspamer et al. and was described in Regulatory Peptides, Vol. 2 (1981), pp. 1-13. Sauvagine has the formula:

$$\text{pGlu-Gly-Pro-Pro-Ile-Ser-Ile-Asp-Leu-Ser-Leu-Glu-Leu-Leu-Arg-Lys-Met-Ile-Glu-Ile-Glu-Lys-Gln-Glu-Lys-Glu-Lys-Gln-Gln-Ala-Ala-Asn-Asn-Arg-Leu-Leu-Leu-Asp-Thr-Ile-NH}_2.$$

Sauvagine and oCRF have been reported to have biological activity in lowering blood pressure in mammals and in stimulating the secretion of ACTH and $\beta$-endorphin.

Rat CRF(rCRF) was later isolated, purified and characterized as a hentetracontapeptide having the formula:

$$\text{H-Ser-Glu-Glu-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Leu-Met-Glu-Ile-Ile-NH}_2.$$

The formula of human CRF has since been determined to be the same as that of rCRF.

Urotensin I (US) has been isolated from urophyses of Catostomus commersoni(white sucker), purified and characterized as a polypeptide having the formula:

$$\text{H-Asn-Asp-Asp-Pro-Pro-Ile-Ser-Ile-Asp-Leu-Thr-Phe-His-Leu-Leu-Arg-Asn-Met-Ile-Glu-Met-Ala-Arg-Ile-Glu-Asn-Glu-Arg-Glu-Gln-Ala-Gly-Leu-Asn-Arg-Lys-Tyr-Leu-Asp-Glu-Val-NH}_2,$$

which is referred to as white sucker urotensin. The purification and characterization are described in an article by Lederis et al., Science Vol. 218, No. 4568, 162-164 (Oct. 8, 1982). A homolog of white sucker

2

urotensin having the following formula:

```
H-Ser-Glu-Glu-Pro-Pro-Met-Ser-Ile-Asp-Leu-Thr-Phe-His-Met-
Leu-Arg-Asn-Met-Ile-His-Arg-Ala-Lys-Met-Glu-Gly-Glu-Arg-
Glu-Gln-Ala-Leu-Ile-Asn-Arg-Asn-Leu-Leu-Asp-Glu-Val-NH₂,
```

was later isolated from the urophyses of Hippoglossoides Elassodon or Flathead Sole, sometimes referred to as Maggy. Synthetic UIs have been found to stimulate ACTH and $\beta$-endorphin activies in vitro and in vivo and to substantially lower blood pressure for an extended time period.

Fish CRF(fCRF) has now been found to be a hentetracontapeptide having the formula:

```
                                          H-Ser-Glu-Glu-
Pro-Pro-Ile-Ser-Leu-Asp-Leu-Thr-Phe-His-Leu-Leu-Arg-Glu-
Val-Leu-Glu-Met-Ala-Arg-Ala-Glu-Gln-Leu-Ala-Gln-Gln-Ala-
His-Ser-Asn-Arg-Lys-Met-Met-Glu-Ile-Phe-NH₂.
```

It may alternatively be referred to as fish Amunine.

This synthesis of the 41-residue peptide has been completed. Synthetic fCRF stimulates ACTH and $\beta$-endorphin activies in vitro and in vivo and substantially lowers blood pressure for an extended time period. As a result, synthetic fCRF is now available in substantially pure form (i.e. substantially free of the remainder of a crude biological extract or of related synthetic replicates), and synthetic compounds having a purity of at least about 5%, which is substantially higher in purity than the naturally occurring peptide, are considered to have utility for such treatment applications. A purity of at least about 95% or higher is practically obtainable and would likely be used for clinical testing.

These peptides can be produced recombinantly, and the invention provides DNA coding for the peptide sequence in the form of:

```
                                  TCG GAG GAG CCG CCG ATT TCC CTG
GAT CTG ACC TTT CAT CTG CTA CGA GAA GTA CTA GAG ATG GCC
AGA GCC GAA CAA TTG GCC CAG CAA GCT CAC AGC AAC CGC AAA
ATG ATG GAA ATA TTC GGG and also in the form of: TCG GAG
GAG CCG CCG ATT TCC CTG GAT CTG ACC TTT CAT CTG CTA CGA
GAA GTA CTA GAG ATG GCC AGA GCC GAA CAA TTG GCC CAG CAA
GCT CAC AGC AAC CGC AAA ATG ATG GAA ATA TTC.
```

Pharmaceutical compositions in accordance with the invention include fCRF, or a nontoxic addition salt thereof, dispersed in a pharmaceutically or veterinarily acceptable liquid or solid carrier. The administration of such peptides or pharmaceutically or veterinarily acceptable addition salts thereof to animals, including mammals and particularly humans, in accordance with the invention, may be carried out for the regulation of secretion of ACTH, $\beta$-endorphin, $\beta$-lipotropin, other products of the pro-opiomelanocortin gene and corticosterone and/or for the lowering of blood pressure and/or for affecting mood, behavioral and gastrointestinal functions and autonomic nervous system activities. Furthermore, it may also be practical to use fCRF for the evaluation of the status of pituitary, cardiovascular, gastrointestinal or central nervous system functions.

The nomenclature used to define the peptides is that specified by Schroder & Lubke, "The Peptides", Academic Press (1965) wherein, in accordance with conventional representation, the amino group appears to the left and the carboxyl group to the right. The standard 3-letter abbreviations to identify the alpha-amino acid residues, and where the amino acid residue has isomeric forms, it is the L-form of the amino acid that is represented unless otherwise expressly indicated, e.g. Ser = L-serine.

The invention provides analogs of CRF having the following Formula (I):

$$Y-R_1-Pro-Pro-Ile-Ser-Leu-Asp-$$
$$Leu-Thr-Phe-His-Leu-Leu-Arg-Glu-Val-Leu-Glu-Met-Ala-Arg-$$
$$Ala-Glu-Gln-Leu-Ala-Gln-Gln-Ala-His-Ser-Asn-Arg-Lys-Met-$$
$$Met-Glu-Ile-Phe-NH_2,$$

wherein Y is an acyl group having 7 or less carbon atoms, preferably acetyl(Ac) or is hydrogen; $R_1$ is Ser-Glu-Glu or Glu-Glu or Glu or des$R_1$; or a nontoxic addition salt thereof.

The peptides are synthesized by a suitable method, such as by exclusively solid-phase techniques, by partial solid-phase techniques, by fragment condensation or by classical solution addition. fCRF may also be synthesized by recently developed recombinant DNA techniques. Synthesis by the use of recombinant DNA techniques, for purposes of this application, should be understood to include the suitable employment of a structural gene coding for fCRF, as is well known in the art at the present time. For example, a suitable structural gene is set forth in Table 1 hereinafter, and an entire gene segment coding for the precursor of fCRF is disclosed in Y. Okawara et al., P.N.A.S. (USA) 85, 8439-8443, Nov. 1988. The synthetic fCRF peptide may be obtained by transforming a microorganism using an expression vector including a promoter and operator together with such structural gene and causing such transformed microorganism to express the fCRF peptide.

## TABLE 1

```
Ser-Glu-Glu-Pro-Pro-Ile-Ser-Leu-Asp-Leu-
TCG GAG GAG CCG CCG ATT TCC CTG GAT CTG

Thr-Phe-His-Leu-Leu-Arg-Glu-Val-Leu-Glu-
ACC TTT CAT CTG CTA CGA GAA GTA CTA GAG

Met-Ala-Arg-Ala-Glu-Gln-Leu-Ala-Gln-Gln-
ATG GCC AGA GCC GAA CAA TTG GCC CAG CAA

Ala-His-Ser-Asn-Arg-Lys-Met-Met-Glu-Ile-
GCT CAC AGC AAC CGC AAA ATG ATG GAA ATA

Phe-NH2
TTC GGG
```

Common to chemical syntheses of peptides is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with various of these residues having side-chain protecting groups.

Thus, the present invention provides intermediates of the Formula (II):

$$X^1-Ser(X^2)-Glu(X^5)-Glu(X^5)-Pro-Pro-Ile-$$
$$Ser(X^2)-Leu-Asp(X^5)-Leu-Thr(X^2)-Phe-His(X)-Leu-Leu-$$
$$Arg(X^3)-Glu(X^5)-Val-Leu-Glu(X^5)-Met-Ala-Arg(X^3)-Ala-$$
$$Glu(X^5)-Gln(X^4)-Leu-Ala-Gln(X^4)-Gln(X^4)-Ala-His(X)-$$
$$Ser(X^2)-Asn(X^4)-Arg(X^3)-Lys(X^6)-Met-Met-Glu(X^5)-Ile-$$
$$Phe-X^7$$

(or suitably N-terminally shortened versions thereof) wherein:

$X^1$ is either hydrogen or an alpha-amino protecting group. The alpha-amino protecting groups contemplated by $X^1$ are those known to be useful in the art in the step-wise synthesis of polypeptides. Among the classes of alpha-amino protecting groups covered by $X^1$ are (1) acyl-type protecting groups, such as formyl, acrylyl(Acr), benzoyl(Bz) and acetyl(Ac) which are preferably used only at the N-terminal; (2) aromatic urethan-type protecting groups, such as benzyloxycarbonyl(Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethoxycarbonyl, isopropyloxycarbonyl, ethoxycarbonyl, allyloxycarbonyl; (4) cycloalkyl urethan-type protecting groups, such as fluorenylmethyloxycarbonyl(FMOC), cyclopentyloxycarbonyl, adamantyloxycarbonyl,and cyclohexyloxycarbonyl; and (5) thiourethan-type protecting groups, such as phenylthiocarbonyl. The preferred alpha-amino protecting group is BOC.

$X^2$ is hydrogen or a protecting group for the hydroxyl group of Thr and Ser and is preferably selected from the class consisting of acetyl(Ac), benzoyl(Bz), tert-butyl, triphenylmethyl(trityl), tetrahydropyranyl, benzyl ether(Bzl) and 2,6-dichlorobenzyl (DCB). The most preferred protecting group is Bzl.

$X^3$ is a protecting group for the guanidino group of Arg preferably selected from the class consisting of nitro, p-toluenesulfonyl(Tos), Z, adamantyloxycarbonyl and BOC, or is hydrogen. Tos is most preferred.

$X^4$ is hydrogen or a protecting group, preferably xanthyl(Xan), for the amido group of Asn or Gln.

$X^5$ is hydrogen or an ester-forming protecting group for the $\beta$- or $\gamma$-carboxyl group of Asp or Glu, preferably selected from the class consisting of benzyl, 2,6-dichlorobenzyl, methyl, ethyl and t-butyl ester. OBzl is most preferred.

$X^5$ is hydrogen or a protecting group for the side chain amino substituent of Lys or Orn. Illustrative of suitable side chain amino protecting groups are Z, 2-chlorobenzyloxycarbonyl(2-Cl-Z), Tos, t-amyloxycarbonyl(Aoc), BOC and aromatic or aliphatic urethan-type protecting groups as specified hereinbefore.

X is hydrogen or a protecting group for the imidazole nitrogen of His such as Tos or 2,4-dinitrophenyl-(DNP). The sulfur of Met may be protected, if desired, with oxygen.

The selection of a side chain amino protecting group is not critical except that it should must be one which is not removed during deprotection of the alpha-amino groups during the synthesis. Hence, the alpha-amino protecting group and the side chain amino protecting group cannot be the same.

$X^7$ is $NH_2$, a protecting group such as an ester or an anchoring bond used in solid phase synthesis for linking to a solid resin support, preferably one represented by the formulae:

-NH-benzhydrylamine (BHA) resin support and

-NH-paramethylbenzhydrylamine (MBHA) resin support.

Cleavage from a BHA or MBHA resin directly gives the CRF analog amide. By employing a methyl-derivative of such a resin, a methyl-substituted amide can be created.

In the formula for the intermediate, at least one of X, $X^1$, $X^2$, $X^3$, $X^4$, $X^5$ and $X^5$ is a protecting group. In selecting a particular side chain protecting group to be used in the synthesis of the peptides, the following rules are followed: (a) the protecting group should be stable to the reagent and under the reaction conditions selected for removing the alpha-amino protecting group at each step of the synthesis, (b) the protecting group should retain its protecting properties and not be split off under coupling conditions and (c) the side chain protecting group must be removable, upon the completion of the synthesis containing the desired amino acid sequence, under reaction conditions that will not alter the peptide chain.

For the acyl group at the N-terminus, which is represented by Y, acetyl, formyl, acrylyl and benzoyl are preferred.

Thus, illustrated herein is a process for the manufacture of compounds defined by the Formula (I) comprising (a) forming a peptide having at least one protective group and having the Formula (II) wherein:

X, $X^1$, $X^2$, $X^3$, $X^4$, $X^5$ and $X^5$ are each either hydrogen or a protective group, and $X^7$ is either a protective group or an anchoring bond to resin support or OH or $NH_2$ and (b) splitting off the protective group or groups or anchoring bond from said peptide of the Formula (II) and (c) if desired, converting a resulting peptide into a nontoxic addition salt thereof.

When the peptides are prepared by chemical synthesis, they are preferably prepared using solid phase synthesis, such as that described by Merrifield, J. Am. Chem. Soc., 85, p 2149 (1964), although other equivalent chemical syntheses known in the art can also be used as previously mentioned. Solid-phase synthesis is commenced from the C-terminus of the peptide by coupling a protected alpha-amino acid to a suitable resin as generally set forth in U.S. Patent No. 4,244,946 issued Jan. 21, 1981 to Rivier et al. Such a starting material for fCRF can be prepared by attaching alpha-amino-protected Phe to a BHA resin.

Phe protected by BOC is coupled to the BHA resin using methylene chloride and dimethylformamide (DMF). Following the coupling of BOC-Phe to the resin support, the alpha-amino protecting group is removed, as by using trifluoroacetic acid(TFA) in methylene chloride, TFA alone or with HCl in dioxane. Preferably 50 volume % TFA in methylene chloride is used with 0-5 weight % 1,2 ethanedithiol. The deprotection is carried out at a temperature between about 0°C. and room temperature. Other standard cleaving reagents and conditions for removal of specific alpha-amino protecting groups may be used as described in Schroder & Lubke, "The Peptides", I pp 72-75 (Academic Press l965).

After removal of the alpha-amino protecting group of Ile, the remaining alpha-amino- and side chain-protected amino acids are coupled step-wise in the desired order to obtain the intermediate compound defined hereinbefore. As an alternative to adding each amino acid separately in the synthesis, some of them may be coupled to one another prior to addition to the solid phase reactor. The selection of an appropriate coupling reagent is within the skill of the art. Particularly suitable as coupling reagents are N,N'-dicyclohexyl carbodiimide(DCCI) and N,N'-diisopropyl carbodiimide(DICI).

The activating reagents used in the solid phase synthesis of the peptides are well known in the peptide art. Examples of suitable activating reagents are carbodiimides, such as N,N'-diisopropyl carbodiimide and N-ethyl-N'-(3-dimethylaminopropyl)carbodiimide. Other activating reagents and their use in peptide coupling are described by Schroder & Lubke, supra, in Chapter III and by Kapoor, J. Phar. Sci., 59, pp I-27 (l970).

Each protected amino acid or amino acid sequence is introduced into the solid phase reactor in about a fourfold excess, and the coupling is carried out in a medium of dimethylformamide(DMF):$CH_2Cl_2$ (l:l) or in DMF or $CH_2Cl_2$ alone. In instances where the coupling is carried out manually, the success of the coupling reaction at each stage of the synthesis is monitored by the ninhydrin reaction, as described by E. Kaiser et al., Anal. Biochem. 34, 595 (1970). In cases where incomplete coupling occurs, the coupling procedure is repeated before removal of the alpha-amino protecting group prior to the coupling of the next amino acid. The coupling reactions can be performed automatically, as on a Beckman 990 automatic synthesizer, using a program such as that reported in Rivier et al., Biopolymers, 1978, 17, pp.1927-1938.

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups X, $X^2$, $X^3$, $X^4$, $X^5$ and $X^5$ and the alpha-amino protecting group $X^1$ (unless it is an acyl group which is intended to be present in the final peptide), to obtain the peptide. When using hydrogen fluoride for cleaving, anisole or cresole and methylethyl sulfide are included in the reaction vessel as scavengers. Because Met is present in the sequence, the BOC protecting group is preferably cleaved with trifluoroacetic acid(TFA)/ethanedithiol prior to cleaving the peptide from the resin to eliminate S-alkylation.

The following Example sets forth the preferred method for synthesizing fCRF analogs by the solid-phase technique.

## EXAMPLE I

The synthesis of fCRF having the formula:

```
H-Ser-Glu-Glu-Pro-Pro-Ile-Ser-Leu-Asp-Leu-Thr-Phe-His-Leu-
Leu-Arg-Glu-Val-Leu-Glu-Met-Ala-Arg-Ala-Glu-Gln-Leu-Ala-
Gln-Gln-Ala-His-Ser-Asn-Arg-Lys-Met-Met-Glu-Ile-Phe-NH₂
```

is conducted in a stepwise manner on a MBHA hydrochloride resin, such as available from Bachem, Inc., having a substitution range of about 0.1 to 0.5 mmoles/gm. resin. The synthesis is performed on an automatic Beckman 990B peptide synthesizer using a suitable program, preferably as follows:

| STEP | REAGENTS AND OPERATIONS | MIX TIMES MIN. |
|---|---|---|
| 1 | $CH_2Cl_2$ wash-80 ml. (2 times) | 3 |
| 2 | Methanol(MeOH) wash-30 ml. (2 times) | 3 |
| 3 | $CH_2Cl_2$ wash-80 ml. (3 times) | 3 |
| 4 | 50 percent TFA plus 5 percent 1,2-ethanedithiol in $CH_2Cl_2$-70 ml. (2 times) | 12 |
| 5 | Isopropanol wash-80 ml. (2 times) | 3 |
| 6 | TEA 12.5 percent in $CH_2Cl_2$-70 ml. (2 times) | 5 |
| 7 | MeOH wash-40 ml. (2 times) | 2 |
| 8 | $CH_2Cl_2$ wash-80 ml. (3 times) | 3 |
| 9 | Boc-amino acid (10 mmoles) in 30 ml. of either DMF or $CH_2Cl_2$, depending upon the solubility of the particular protected amino acid, (1 time) plus DCCI (10 mmoles) in $CH_2Cl_2$ | 30-300 |

Coupling of BOC-Phe results in the substitution of about 0.35 mmol. Phe per gram of resin. All solvents that are used are carefully degassed, preferably by sparging with an inert gas, e.g. helium or nitrogen, to insure the absence of oxygen that might undesirably oxidize the sulfur of the Met residue.

After deprotection and neutralization, the peptide chain is built step-by-step on the resin. Generally, one to two mmol. of BOC-protected amino acid in methylene chloride is used per gram of resin, plus one equivalent of 2 molar DCCI in methylene chloride, for two hours. When BOC-Arg(Tos) is being coupled, a mixture of 50% DMF and methylene chloride is used. Bzl is used as the hydroxyl side-chain protecting group for Ser and Thr. P-nitrophenyl ester(ONp) is used to activate the carboxyl end of Asn or Gln, and for example, BOC-Asn(ONp) is coupled overnight using one equivalent of HOBt in a 50% mixture of DMF and methylene chloride. The amido group of Asn or Gln is protected by Xan when DCCI coupling is used instead of the active ester method. 2-Cl-Z is used as the protecting group for the Lys side chain. Tos is used to protect the guanidino group of Arg and the imidazole group of His, and the side chain carboxyl group of Glu or Asp is protected by OBzl. At the end of the synthesis, the following composition is obtained

```
BOC-Ser(Bzl)-Glu(OBzl)-Glu(OBzl)-Pro-Pro-Ile-
Ser(Bzl)-Leu-Asp(OBzl)-Leu-Thr(Bzl)-Phe-His(Tos)-Leu-Leu-
Arg(Tos)-Glu(OBzl)-Val-Leu-Glu(OBzl)-Met-Ala-Arg(Tos)-Ala-
Glu(OBzl)-Gln(Xan)-Leu-Ala-Gln(Xan)-Gln(Xan)-Ala-His(Tos)-
Ser(Bzl)-Asn(Xan)-Arg(Tos)-Lys(2-Cl-Z)-Met-Met-Glu(OBzl)-
Ile-Phe-resin support.
```

Xan may have been partially or totally removed by TFA treatment used to deblock the alpha-amino protecting group.

In order to cleave and deprotect the resulting protected peptide-resin, it is treated with 1.5 ml. anisole, 0.5 ml. of methylethylsulfide and 15 ml. hydrogen fluoride (HF) per gram of peptide-resin, first at -20°C. for 20 min. and then at 0°C. for one-half hour. After elimination of the HF under high vacuum, the resin-peptide is washed alternately with dry diethyl ether and chloroform, and the peptides are then extracted with degassed 2N aqueous acetic acid and separated from the resin by filtration.

The peptide is purified by gel permeation followed by semi-preparative HPLC as described in Rivier et al., Peptides: Structure and Biological Function (1979) pp. 125-128, and Rivier et al., J. Chromatography - (1983). The chromatographic fractions are carefully monitored by HPLC, and only the fractions showing substantial purity were pooled.

Specific optical rotation of the rCRF peptide, which was synthesized and purified in the foregoing manner, was measured on a Perkin Elmer Model 141 as $[\alpha]_D$ = -30.5 ± 1.0 (c = 0.2 in 50% acetic acid) (with correction for the presence of $H_2O$ and TFA) and had a purity of about 95%. To check whether the precise sequence was achieved, the fCRF peptide was hydrolyzed in sealed evacuated tubes containing constant boiling HCl, 3 $\mu$l of thioglycol/ml. and 1 nmol of Nle (as an internal standard) for 9 hours at 140°C. Amino acid analyses of the hydrolysates using a Beckman 121 MB amino acid analyzer showed the expected amino acid ratios which confirmed that the 41-residue peptide structure had been obtained.

The peptide stimulates the secretion of ACTH and $\beta$-endorphin by cultured rat pituitary cells and also stimulates ACTH and $\beta$-END-LI secretion <u>in</u> <u>vivo</u> in several rat preparations. In the dog, the peptide produces sustained depression of the blood pressure. The peptide also causes a marked fall in blood pressure in the rat and stimulates the secretion of ACTH by teleost fish pituitaries.

**EXAMPLE II**

The peptide having the formula:

Ac-Ser-Glu-Glu-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Met-Met-Glu-Ile-Phe-NH$_2$

is synthesized in the same manner as in Example I but in addition subjecting the N-terminus to acetylation by treatment with acetic anhydride after removal of the BOC-protecting group. Testing shows that it likewise stimulates the secretion of ACTH and $\beta$-END-LI and causes vasodilatory-hypotensive activity, including lowering of systemic blood pressure.

**EXAMPLE III**

The peptide having the formula:

H-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Met-Met-Glu-Ile-Phe-NH$_2$

is synthesized. Testing shows that it has significant mammalian vasodilatory-hypotensive activity, including the lowering of systemic blood pressure and that it stimulates the secretion of ACTH.

fCRF profoundly stimulates the pituitary-adrenalcortical axis and should be useful to stimulate the functions of this axis in some types of patients with low endogenous glucocorticoid production. For example, fCRF should be useful in restoring pituitary-adrenal function in patients having received exogenous glucocorticoid therapy whose pituitary-adrenalcortical functions remain supressed.

Most other regulatory peptides have been found to have effects upon the central nervous system and upon the gastrointestinal tract. Because ACTH and $\beta$-END secretion is the "sine qua non" of mammal's response to stress, it was not surprising that CRF has significant effects on the brain as a mediator of the body's stress response. Accordingly, fCRF should also find application in modifying the mood, learning and behavior of normal and mentally disordered individuals. Because fCRF elevates the levels of ACTH, $\beta$-END, $\beta$-lipotropin, other pro-opiomelanocortin gene products and corticosterone, its administration can be used to induce their effects on the brain and its periphery to thereby influence memory, mood, pain appreciation, etc., and more specifically, alertness, depression and/or anxiety. For example, when administered into the ventricles, fCRF increases activity and improves learning performance in rats and thus may function as a natural stimulant.

fCRF should also be of use for increasing blood flow to the gastrointestinal tract of animals, particularly humans and other mammals. All CRF related peptides have been shown to dialate the mesenteric vascular bed. Also, oCRF inhibits gastric acid production, and fCRF is expected to also be effective in the treatment of gastric ulcers by reducing gastric acid production and/or inhibiting gastrointestinal functions in a mammal.

fCRF or the nontoxic addition salts thereof, combined with a pharmaceutically or veterinarily acceptable carrier to form a pharmaceutical composition, may be administered to animals, including humans and other mammals, either intravenously, subcutaneously, intramuscularly, percutaneously, e.g. intranasally, intracerebrospinally or orally. The peptides should be at least about 90% pure and preferably should have a purity of at least about 98%; however, lower purities are effective and may well be used with mammals other than humans. This purity means that the intended peptide constitutes the stated weight % of all like peptides and peptide fragments present. Administration to humans may be employed by a physician to lower blood pressure or to stimulate endogenous gluco-corticoid production. The required dosage will vary with the particular condition being treated, with the severity of the condition and with the duration of desired treatment.

These peptides may also be used to evaluate hypothalamic pituitary adrenal function in mammals with suspected endocrine or central nervous system pathology by suitable administration followed by monitoring body functions. For example, administration may be used as a diagnostic tool to evaluate Cushing's disease and affective disorders, such as depressive illness.

Such peptides are often administered in the form of pharmaceutically or veterinarily acceptable nontoxic salts, such as acid addition salts or metal complexes, e.g., with zinc, iron, calcium, barium, magnesium, aluminum or the like (which are considered as addition salts for purposes of this application). Illustrative of such acid addition salts are hydrochloride, hydrobromide, sulphate, phosphate, tannate, oxalate, fumarate, gluconate, alginate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered under the guidance of a physician, and pharmaceutical compositions will usually contain the peptide in conjunction with a conventional, pharmaceutically or veterinarily-acceptable carrier. Usually, the dosage will be from about 1 to about 200 micrograms of the peptide per kilogram of the body weight of the host animal. In some instances, treatment of subjects with these peptides can be carried out in lieu of the administration of ACTH or corticosteroids, in such instances a dosage as low as about 10 ng/Kg of body weight may be employed. As used herein all temperatures are °C. and all ratios are by volume. Percentages of liquid materials are also by volume.

Although the invention has been described with regard to its preferred embodiments, which constitute the best mode presently known to the inventors, it should be understood that various changes and modifications as would be obvious to one having the ordinary skill in this art may be made without departing from the scope of the invention which is set forth in the claims appended hereto. For example, instead of the simple amide at the C-terminus, a lower alkyl-substituted amide, e.g. methylamide, ethylamide, etc, may be incorporated. Likewise from one to ten additional amino acid residues can be included at the N-terminus without significantly adversely affecting biological potency. Such peptides are considered as being within the scope of the invention.

Various features of the invention are emphasized in the claims which follow.

## Claims
## Claims for the following Contracting States : AT, BE, CH, DE, FR, GB, IT, LI, LU, NL, SE

1. A polypeptide having the formula:

```
Y-R₁-Pro-Pro-Ile-Ser-Leu-Asp-Leu-Thr-Phe-His-Leu-Leu-
Arg-Glu-Val-Leu-Glu-Met-Ala-Arg-Ala-Glu-Gln-Leu-Ala-Gln-
Gln-Ala-His-Ser-Asn-Arg-Lys-Met-Met-Glu-Ile-Phe-NH₂
```

or a biologically active fragment thereof, wherein Y is an acyl group having 7 or less carbon atoms or hydrogen and $R_1$ is Ser-Glu-Glu or Glu-Glu or Glu or des-$R_1$; or a nontoxic addition salt thereof.

2. A polypeptide according to Claim 1 wherein $R_1$ is Ser-Glu-Glu.

3. A polypsptide according to Claim 1 wherein $R_1$ is Glu.

**4.** A polypeptide according to Claim 1 wherein $R_1$ is des$R_1$.

**5.** A polypeptide according to Claim 1 having formula:

```
         H-Ser-Glu-Glu-Pro-Pro-Ile-Ser-Leu-Asp-Leu-Thr-
    Phe-His-Leu-Leu-Arg-Glu-Val-Leu-Glu-Met-Ala-Arg-Ala-Glu-
    Gln-Leu-Ala-Gln-Gln-Ala-His-Ser-Asn-Arg-Lys-Met-Met-Glu-
    Ile-Phe-NH₂.
```

**6.** A polypeptide in accordance with Claim 1 having the formula:

```
            Ac-Ser-Glu-Glu-Pro-Pro-Ile-Ser-Leu-
    Asp-Leu-Thr-Phe-His-Leu-Leu-Arg-Glu-Val-Leu-Glu-Met-Ala-
    Arg-Ala-Glu-Gln-Leu-Ala-Gln-Gln-Ala-His-Ser-Asn-Arg-Lys-
    Met-Met-Glu-Ile-Phe-NH₂.
```

**7.** A polypeptide in accordance with Claim 1 having the formula:

```
            H-Pro-Pro-Ile-Ser-Leu-Asp-Leu-Thr-
    Phe-His-Leu-Leu-Arg-Glu-Val-Leu-Glu-Met-Ala-Arg-Ala-Glu-
    Gln-Leu-Ala-Gln-Gln-Ala-His-Ser-Asn-Arg-Lys-Met-Met-Glu-
    Ile-Phe-NH₂.
```

**8.** A polypeptide having CRF-associated biological activity and comprised of the amino acid residue sequence set forth and defined in Claim 1.

**9.** A polypeptide as claimed in Claim 8 and consisting of the amino acid sequence set forth and defined in Claim 1 modified by N-terminus addition of from 1 to 10 amino acid residues.

**10.** A non-toxic salt of a polypeptide as claimed in any one of Claims 2 to 9.

**11.** DNA coding for a polypeptide as claimed in any preceding claim.

**12.** DNA coding for the following polypeptide sequence:

```
    Ser-Glu-Glu-Pro-Pro-Ile-Ser-Leu-Asp-Leu-Thr-Phe-His-Leu-
    Leu-Arg-Glu-Val-Leu-Glu-Met-Ala-Arg-Ala-Glu-Gln-Leu-Ala-
    Gln-Gln-Ala-His-Ser-Asn-Arg-Lys-Met-Met-Glu-Ile-Phe.
```

**13.** DNA according to Claim 12 including the following sequence:

```
TCG GAG GAG CCG CCG ATT TCC CTG GAT CTG ACC TTT

CAT CTG CTA CGA GAA GTA CTA GAG ATG GCC AGA GCC GAA CAA

TTG GCC CAG CAA GCT CAC AGC AAC CGC AAA ATG ATG GAA ATA

TTC.
```

**14.** A pharmaceutical composition for regulating vasodilatory-hypotensive activity which comprises an effective amount of a polypeptide according to any one of Claims 1 to 9 or a polypeptide salt according to Claim 1 or Claim 10 in combination with a pharmaceutically acceptable carrier.

**Claims for the following Contracting States : ES, GR**

**1.** A process for the manufacture of compounds defined by the Formula (I):

$$Y-R_1-Pro-Pro-Ile-Ser-Leu-$$
$$Asp-Leu-Thr-Phe-His-Leu-Leu-Arg-Glu-Val-Leu-Glu-Met-Ala-$$
$$Arg-Ala-Glu-Gln-Leu-Ala-Gln-Gln-Ala-His-Ser-Asn-Arg-Lys-$$
$$Met-Met-Glu-Ile-Phe-NH_2$$

or a biologically active fragment thereof, wherein Y is an acyl group having 7 or less carbon atoms or hydrogen and $R_1$ is Ser-Glu-Glu or Glu-Glu or Glu or des-$R_1$; or a nontoxic addition salt thereof, which process comprises (a) forming an intermediate peptide having at least one protective group and having the
Formula (II):

$$X^1-Ser(X^2)-Glu(X^5)-Glu(X^5)-Pro-Pro-Ile-$$
$$Ser(X^2)-Leu-Asp(X^5)-Leu-Thr(X^2)-Phe-His(X)-Leu-Leu-$$
$$Arg(X^3)-Glu(X^5)-Val-Leu-Glu(X^5)-Met-Ala-Arg(X^3)-Ala-$$
$$Glu(X^5)-Gln(X^4)-Leu-Ala-Gln(X^4)-Gln(X^4)-Ala-His(X)-$$
$$Ser(X^2)-Asn(X^4)-Arg(X^3)-Lys(X^6)-Met-Met-Glu(X^5)-Ile-$$
$$Phe-X^7$$

(or suitably N-terminally shortened versions thereof) wherein: X, $X^1$, $X^2$, $X^3$, $X^4$, $X^5$ and $X^5$ are each either hydrogen or aprotective group, and $X^7$ is either a protective group or an anchoring bond to resin support or OH or $NH_2$, (b) splitting off the protective group or groups or anchoring bond from said peptide of the Formula (II) and (c) if desired, converting a resulting peptide into a nontoxic addition salt thereof.

**2.** A process according to Claim 1 wherein $R_1$ is Ser-Glu-Glu.

**3.** A process according to Claim 1 wherein $R_1$ is Glu.

**4.** A process according to Claim 1 wherein $R_1$ is desR_1.

5. A process according to Claim 1 wherein the compound has the formula:

```
                               H-Ser-Glu-Glu-Pro-Pro-Ile-Ser-
Leu-Asp-Leu-Thr-Phe-His-Leu-Leu-Arg-Glu-Val-Leu-Glu-Met-
Ala-Arg-Ala-Glu-Gln-Leu-Ala-Gln-Gln-Ala-His-Ser-Asn-Arg-
Lys-Met-Met-Glu-Ile-Phe-NH₂.
```

6. A process in accordance with Claim 1 wherein the compound has the formula:

```
                               Ac-Ser-Glu-Glu-Pro-Pro-
Ile-Ser-Leu-Asp-Leu-Thr-Phe-His-Leu-Leu-Arg-Glu-Val-Leu-
Glu-Met-Ala-Arg-Ala-Glu-Gln-Leu-Ala-Gln-Gln-Ala-His-Ser-
Asn-Arg-Lys-Met-Met-Glu-Ile-Phe-NH₂.
```

7. A process in accordance with Claim 1 wherein the compound has the formula:

```
                               H-Pro-Pro-Ile-Ser-Leu-
Asp-Leu-Thr-Phe-His-Leu-Leu-Arg-Glu-Val-Leu-Glu-Met-Ala-
Arg-Ala-Glu-Gln-Leu-Ala-Gln-Gln-Ala-His-Ser-Asn-Arg-Lys-
Met-Met-Glu-Ile-Phe-NH₂.
```

8. A process for making a recombinant polypeptide which uses DNA coding for the following polypeptide sequence:

```
                       Ser-Glu-Glu-Pro-Pro-Ile-Ser-Leu-
Asp-Leu-Thr-Phe-His-Leu-Leu-Arg-Glu-Val-Leu-Glu-Met-Ala-
Arg-Ala-Glu-Gln-Leu-Ala-Gln-Gln-Ala-His-Ser-Asn-Arg-Lys-
Met-Met-Glu-Ile-Phe.
```

9. A process according to Claim 8 where said DNA includes the following sequence:

```
                               TCG GAG GAG CCG CCG
ATT TCC CTG GAT CTG ACC TTT CAT CTG CTA CGA GAA GTA CTA
GAG ATG GCC AGA GCC GAA CAA TTG GCC CAG CAA GCT CAC AGC
AAC CGC AAA ATG ATG GAA ATA TTC.
```

**Patentansprüche**
**Patentansprüche für folgende Vertragsstaaten : AT, BE, CH, DE, FR, GB, IT, LI, LU, NL, SE**

1. Polypeptid der Formel:

```
Y-R₁-Pro-Pro-Ile-Ser-Leu-Asp-Leu-Thr-Phe-His-Leu-Leu-Arg-Glu-
Val-Leu-Glu-Met-Ala-Arg-Ala-Glu-Gln-Leu-Ala-Gln-Gln-Ala-His-
Ser-Asn-Arg-Lys-Met-Met-Glu-Ile-Phe-NH₂
```

oder ein biologisch aktives Fragment davon, worin Y eine Acyl-Gruppe mit 7 oder weniger Kohlenstoffatomen oder Wasserstoff ist und $R_1$ Ser-Glu-Glu oder Glu-Glu oder Glu oder des-$R_1$ ist; oder ein nicht toxisches Additions Salz davon.

2. Polypeptid nach Anspruch 1, worin $R_1$ Ser-Glu-Glu ist.

3. Polypepitd nach Anspruch 1, worin $R_1$ Glu ist.

4. Polypeptid nach Anspruch 1, worin $R_1$ des-$R_1$ ist.

5. Polypeptid nach Anspruch 1 der Formel:

```
                                    H-Ser-Glu-Glu-Pro-
Pro-Ile-Ser-Leu-Asp-Leu-Thr-Phe-His-Leu-Leu-Arg-Glu-Val-Leu-
Glu-Met-Ala-Arg-Ala-Glu-Gln-Leu-Ala-Gln-Gln-Ala-His-Ser-Asn-
Arg-Lys-Met-Met-Glu-Ile-Phe-NH₂.
```

6. Polypetid nach Anspruch 1 der Formel:

```
                                    Ac-Ser-Glu-Glu-Pro-
Pro-Ile-Ser-Leu-Asp-Leu-Thr-Phe-His-Leu-Leu-Arg-Glu-Val-Leu-
Glu-Met-Ala-Arg-Ala-Glu-Gln-Leu-Ala-Gln-Gln-Ala-His-Ser-Asn-
Arg-Lys-Met-Met-Glu-Ile-Phe-NH₂.
```

7. Polypeptid nach Anspruch 1 der Formel:

```
                                    H-Pro-Pro-Ile-Ser-
Leu-Asp-Leu-Thr-Phe-His-Leu-Leu-Arg-Glu-Val-Leu-Glu-Met-Ala-
Arg-Ala-Glu-Gln-Leu-Ala-Gln-Gln-Ala-His-Ser-Asn-Arg-Lys-Met-
Met-Glu-Ile-Phe-NH₂.
```

8. Polypeptid mit CRF-assoziierter, biologischer Aktivität und umfaßt in der in Anspruch 1 gezeigten und definierten Aminosäresequenz.

9. Polypeptid nach Anspruch 8 und bestehend aus der in Anspruch 1 gezeigten und definierten Aminosäuresequenz, das durch Anfügen von 1 bis 10 Aminosäuren an den N-Terminus modifiziert ist.

**10.** Nicht-toxisches Salz eines Polypeptids nach einem der Ansprüche 2 bis 9.

**11.** DNA, die ein Polypeptid nach einem der vorhergehenden Ansprüche codiert.

**12.** DNA, die die folgende Polypeptidsequenz codiert:

```
                                                   Ser-Glu-
Glu-Pro-Pro-Ile-Ser-Leu-Asp-Leu-Thr-Phe-His-Leu-Leu-Arg-Glu-
Val-Leu-Glu-Met-Ala-Arg-Ala-Glu-Gln-Leu-Ala-Gln-Gln-Ala-His-
Ser-Asn-Arg-Lys-Met-Met-Glu-Ile-Phe.
```

**13.** DNA, nach Anspruch 12, die die folgende Sequenz einschließt:

```
        TCG  GAG  GAG  CCG  CCG  ATT  TCC  CTG  GAT  CTG  ACC  TTT
CAT  CTG  CTA  CGA  GAA  GTA  CTA  GAG  ATG  GCC  AGA  GCC  GAA  CAA  TTG  GCC
CAG  CAA  GCT  CAC  AGC  AAC  CGC  AAA  ATG  ATG  GAA  ATA  TTC.
```

**14.** Pharmazeutisch Zusammensetzung zur Regulierung vasodilatorisch-hypotensiver Aktivität umfassend eine wirksame Menge eines Polypeptids nach einem der Ansprüche 1 bis 9, oder ein Polypeptid-Salz nach Anspruch 1 oder 10 in Verbindung mit einem pharmazeutisch verträglichen Träger.

**Patentansprüche für folgende Vertragsstaaten : GR, ES**

**1.** Verfahren zur Herstellung von Verbindungen, die durch die Formel (I) definiert sind:

$$Y-R_1-Pro-Pro-Ile-Ser-Leu-Asp-Leu-$$
$$Thr-Phe-His-Leu-Leu-Arg-Glu-Val-Leu-Glu-Met-Ala-Arg-Ala-Glu-$$
$$Gln-Leu-Ala-Gln-Gln-Ala-His-Ser-Asn-Arg-Lys-Met-Met-Glu-Ile-$$
$$Phe-NH_2$$

oder einem biologisch aktiven Fragment davon, worin Y eine Acyl-Gruppe mit 7 oder weniger Kolenstoffatomen oder Wasserstoff ist, und $R_1$ Ser-Glu-Glu oder Glu-Glu oder Glu oder des-$R_1$ ist; oder ein nicht-toxisches Additions-Salz davon, wobei das Verfahren umfaßt, (a) Bildung eines Zwischen-Peptids mit wenigstens einer Schutzgruppe und der Formel (II):

$$X^1-$$
$$Ser(X^2)-Glu(X^5)-Glu(X^5)-Pro-Pro-Ile-Ser(X^2)-Leu-Asp(X^5)-Leu-$$
$$Thr(X^2)-Phe-His(X)-Leu-Leu-Arg(X^3)-Glu(X^5)-Val-Leu-Glu(X^5)-Met-$$
$$Ala-Arg(X^3)-Ala-Glu(X^5)Gln(X^4)-Leu-Ala-Gln(X^4)-Gln(X^4)-Ala-$$
$$His(X)-Ser(X^2)-Asn(X^4)-Arg(X^3)-Lys(X^6)-Met-Met-Glu(X^5)-Ile-Phe-$$
$$(X^7)$$

(oder geeignete am N-Terminus gekürzte Versionen davon), worin X, $X^1$, $X^2$, $X^3$, $X^4$, $X^5$ und $X^5$ jeweils ein Wasserstoff oder eine Schutzgruppe sind, und $X^7$ entweder eine Schutzgruppe oder eine verankernde Bindung an ein Harz-Trägermaterial ist oder OH oder $NH_2$, (b) Abspalten der Schutz-Gruppe oder der Gruppen oder der verankernden Bindung von dem Peptid der Formel (II) und (c) wenn gewünscht, Umwandeln eines so erhaltenen Peptids in ein nicht-toxisches Salz davon.

**2.** Verfahren nach Anspruch 1 worin $R_1$ Ser-Glu-Glu ist.

**3.** Verfahren nach Anspruch 1 worin $R_1$ Glu ist.

**4.** Verfahren nach Anspruch 1 worin $R_1$ des-$R_1$ ist.

**5.** Verfahren nach Anspruch 1 worin die Verbindung die Formel hat:

```
H-Ser-Glu-Glu-Pro-Pro-Ile-Ser-Leu-Asp-Leu-Thr-Phe-His-Leu-
Leu-Arg-Glu-Val-Leu-Glu-Met-Ala-Arg-Ala-Glu-Gln-Leu-Ala-Gln-
Gln-Ala-His-Ser-Asn-Arg-Lys-Met-Met-Glu-Ile-Phe-NH2.
```

**6.** Verfahren nach Anspruch 1 worin die Verbindung die Formel hat:

```
Ac-Ser-Glu-Glu-Pro-Pro-Ile-Ser-Leu-Asp-Leu-Thr-Phe-His-
Leu-Leu-Arg-Glu-Val-Leu-Glu-Met-Ala-Arg-Ala-Glu-Gln-Leu-Ala-
Gln-Gln-Ala-His-Ser-Asn-Arg-Lys-Met-Met-Glu-Ile-Phe-NH2.
```

**7.** Verfahren nach Anspruch 1 worin die Verbindung die Formel hat:

```
H-Pro-Pro-Ile-Ser-Leu-Asp-Leu-Thr-Phe-His-Leu-Leu-Arg-Glu-
Val-Leu-Glu-Met-Ala-Arg-Ala-Glu-Gln-Leu-Ala-Gln-Gln-Ala-His-
Ser-Asn-Arg-Lys-Met-Met-Glu-Ile-Phe-NH2.
```

**8.** Verfahren zur Herstellung eines recombinanten Polypeptids, welches DNA verwendet, die die folgende Peptidsequenz codiert:

```
Ser-Glu-Glu-Pro-Pro-Ile-Ser-Leu-Asp-Leu-Thr-Phe-His-Leu-Leu-
Arg-Glu-Val-Leu-Glu-Met-Ala-Arg-Ala-Glu-Gln-Leu-Ala-Gln-Gln-
Ala-His-Ser-Asn-Arg-Lys-Met-Met-Glu-Ile-Phe.
```

**9.** Verfahren nach Anspruch 8 bei dem die DNA die folgende Sequenz einschließt:

```
                TCG GAG GAG CCG CCG ATT TCC CTG GAT CTG
ACC TTT CAT CTG CTA CGA GAA GTA CTA GAG ATG GCC AGA GCC GAA CAA
TTG GCC CAG CAA GCT CAC AGC AAC CGC AAA ATG ATG GAA ATA TTC.
```

15

**Revendications**
**Revendications pour les Etats contractants suivants : AT, BE, CH, DE, FR, GB, IT, LI, LU, NL, SE**

**1.** Polypeptide de formule :

```
Y-R1-Pro-Pro-Ile-Ser-Leu-Asp-Leu-Thr-Phe-His-Leu-Leu-Arg-
Glu-Val-Leu-Glu-Met-Ala-Arg-Ala-Glu-Gln-Leu-Ala-Gln-Gln-
Ala-His-Ser-Asn-Arg-Lys-Met-Met-Glu-Ile-Phe-NH2
```

ou un fragment de celui-ci biologiquement actif, dans lequel Y est un groupement acyle de 7 atomes de carbone au plus ou un hydrogène et $R_1$ est le Ser-Glu-Glu ou le Glu-Glu ou le Glu ou dés-$R_1$ ; ou un sel d'addition de celui-ci non toxique.

**2.** Polypeptide selon la revendication 1, dans lequel $R_1$ est le Ser-Glu-Glu.

**3.** Polypeptide selon la revendication 1, dans lhequel $R_1$ est le Glu.

**4.** Polypeptide selon la revendication 1, dans lequel $R_1$ est dés$R_1$.

**5.** Polypeptide selon la revendication 1 de formule :

```
H-Ser-Glu-Glu-Pro-Pro-Ile-Ser-Leu-Asp-Leu-Thr-Phe-His-
Leu-Leu-Arg-Glu-Val-Leu-Glu-Met-Ala-Arg-Ala-Glu-Gln-Leu-
Ala-Gln-Gln-Ala-His-Ser-Asn-Arg-Lys-Met-Met-Glu-Ile-Phe-
NH2.
```

**6.** Polypeptide conformément à la revendication 1 de formule :

```
            Ac-Ser-Glu-Glu-Pro-Pro-Ile-ser-Leu-Asp-Leu-
Thr-Phe-His-Leu-Leu-Arg-Glu-Val-Leu-Glu-Met-Ala-Arg-Ala-
Glu-Gln-Leu-Ala-Gln-Gln-Ala-His-Ser-Asn-Arg-Lys-Met-Met-
Glu-Ile-Phe-NH2.
```

**7.** Polypeptide conformément à la revendication 1 de formule :

```
H-Pro-Pro-Ile-Ser-Leu-Asp-Leu-Thr-Phe-His-Leu-
```

```
Leu-Arg-Glu-Val-Leu-Glu-Met-Ala-Arg-Ala-Glu-Gln-Leu-Ala-
Gln-Gln-Ala-His-Ser-Asn-Arg-Lys-Met-Met-Glu-Ile-Phe-NH2.
```

**8.** Polypeptide possédant l'activité biologique CRF-associée et comportant la séquence résidu d 'acide aminé énoncée et définie dans la revendication 1.

16

**9.** Polypeptide comme le mentionne la revendication 8 et constitué par la séquence d'acide aminé énoncée et définie dans la revendication 1 modifiée par l'addition à N-terminus de 1 à 10 résidus d'acide aminé.

**10.** Sel non toxique d'un polypeptide comme le mentionne n'importe laquelle des revendications 2 à 9.

**11.** Codage de l'ADN pour un polypeptide comme le mentionne n'importe quelle revendication précédente.

**12.** Codage de l 'ADN pour la séquence du polypeptide suivant :

```
            Ser-Glu-Glu-Pro-Pro-Ile-Ser-Leu-Asp-Leu-Thr-
    Phe-His-Leu-Leu-Arg-Glu-Val-Leu-Glu-Met-Ala-Arg-Ala-Glu-
    Gln-Leu-Ala-Gln-Gln-Ala-His-Ser-Asn-Arg-Lys-Met-Met-Glu-
    Ile-Phe.
```

**13.** ADN selon la revendication 12 constitué par la séquence suivante :

```
                TCG  GAG  GAG  CCG  CCG  ATT  TCC  CTG  GAT
    CTG  ACC  TTT  CAT  CTG  CTA  CGA  GAA  GTA  CTA  GAG  ATG  GCC  AGA
    GCC  GAA  CAA  TTG  GCC  CAG  CAA  GCT  CAC  AGC  AAC  CGC  AAA  ATG
    ATG  GAA  ATA  TTC.
```

**14.** Composition pharmaceutique pour réguler l'activité vasodilatatrice-hypotensive, laquelle comporte une quantité efficace d'un polypeptide selon n'importe laquelle des revendications 1 à 9 ou un sel de polypeptide selon la revendication 1 ou 10, combiné avec un agent pharmaceutique acceptable.

**Revendications pour les Etats contractants suivants : GR, ES**

**1.** Procédé pour la fabrication de composés définis par la formule (I) :

$$Y-R_1-Pro-Pro-Ile-Ser-Leu-Asp-Leu-Thr-$$
$$Phe-His-Leu-Leu-Arg-Glu-Val-Leu-Glu-Met-Ala-Arg-Ala-Glu-$$
$$Gln-Leu-Ala-Gln-Gln-Ala-His-Ser-Asn-Arg-Lys-Met-Met-Glu-$$
$$Ile-Phe-NH_2$$

ou un fragment de celui-ci biologiquement actif, dans lequel Y est un groupement acyle de 7 atomes de carbone au plus ou un hydrogène et $R_1$ le Ser-Glu-Glu ou le Glu-Glu ou le Glu ou dés-$R_1$ ; ou un sel d'addition de celui-ci non toxique, lequel procédé comprend (a) la formation d'un peptide intermédiaire possédant au moins un groupement protecteur et de formule (II) :

$$X^1-Ser(X^2)-$$
$$Glu(X^5)-Glu(X^5)-Pro-Pro-Ile-Ser(X^2)-Leu-Asp(X^5)-Leu-$$
$$Thr(X^2)-Phe-His(X)-Leu-Leu-Arg(X^3)-Glu(X^5)-Val-Leu-$$
$$Glu(X^5)-Met-Ala-Arg(X^3)-Ala-Glu(X^5)-Gln(X^4)-Leu-Ala-$$
$$Gln(X^4)-Gln(X^4)-Ala-His(X)-Ser(X^2)-Asn(X^4)-Arg(X^3)-$$
$$Lys(X^6)-Met-Met-Glu(X^5)-Ile-Phe-X^7$$

17

(ou leurs versions bien raccourcies de N-terminal) dans lequel : X, $X^1$, $X^2$, $X^3$, $X^4$, $X^5$ et $X^5$ représentent chacun un hydrogène ou un groupement protecteur, et $X^7$ soit un groupement protecteur soit une liaison de fixation pour le support de résine soit OH soit $NH_2$, (b) le détachement du ou des groupements protecteurs ou de la liaison de fixation dudit peptide de formule (II), et (c) si désiré, la transformation d'un peptide résultant en un sel d'addition de celui-ci non toxique.

2. Procédé selon la revendication 1, dans lequel $R_1$ est le Ser-Glu-Glu.

3. Procédé selon la revendication 1, dans lequel $R_1$ est le Glu.

4. Procédé selon la revendication 1, dans lequel $R_1$ est désR$_1$.

5. Procédé selon la revndicaticn 1, dans lequel le composé a la formule :

H-Ser-Glu-Glu-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Met-Met-Glu-Ile-Phe-NH$_2$.

6. Procédé conformément à la revendication 1, dans lequel le composé a la formule :

Ac-Ser-Glu-Glu-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Met-Met-Glu-Ile-Phe-Nh$_2$.

7. Procédé conformément à la revendication 1, dans lequel le composé a la formule :

H-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Met-Met-Glu-Ile-Phe-NH$_2$.

8. Procédé pour fabriquer un polypeptide recombinant, qui utilise le codage de l'ADN pour la séquence du polypeptide suivant :

Ser-Glu-Glu-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Met-Met-Glu-Ile-Phe.

**9.** Procédé selon la revendication 8 où ledit ADN comporte la séquence suivante :

```
                                     TCG GAG GAG CCG CCG ATT
TCC CTG GAT CTG ACC TTT CAT CTG CTA CGA GAA GTA CTA GAG
ATG GCC AGA GCC GAA CAA TTG GCC CAG CAA GCT CAC AGC AAC
CGC AAA ATG ATG GAA ATA TTC.
```